# EUROPEAN PATENT APPLICATION

(11) **EP 2 484 335 A1**
(43) Date of publication of application: **08.08.2012**
(21) Application number: 11380078.3
(22) Date of filing: 10.10.2011
(51) Int. Cl.: A61K 8/06, A61K 8/34, A61K 8/92, A61K 8/97, A61Q 9/02, A61Q 19/00

(54) **Shaving cream for the treatment of acne and the irritations and infections caused by acne**

(30) Priority: 21.10.2010 ES 201031546
(71) Applicant: Perez, Federico Luiz, Andorra La Vella (ES)
(72) Inventor: Perez, Federico Luiz, Andorra La Vella (ES)
(74) Representative: Espiell Volart, Eduardo Maria

(57) **Abstract**

This cream is composed of a mixture of a solid part and a liquid part, with a proportion of 1 gram of solid part for every 2.65 millilitres of liquid part; where the solid part is composed of sweet almond powdered soap, rich in moisturising glycerine; and the liquid part is composed of a combination of the following components: orchid gel, almond oil, high-foam, soap-free baby bath gel, ecological chamomile tea, alcohol-free eau de cologne, and 70° baby alcohol boricated to saturation.

## Description

### OBJECT OF THE INVENTION

The present invention relates to a cream for the treatment of acne and the irritations and infections produced by the different varieties of acne, which presents characteristics aimed at allowing the use thereof as a shaving cream or even also as a face mask.

### FIELD OF THE INVENTION

This invention has application within the industry dedicated to the manufacturing of products for topical use, for the treatment of acne and the irritations and infections produced by the different varieties of acne.

### BACKGROUND OF THE INVENTION

Acne is a skin condition caused by a blockage of sebaceous secretion and inflammatory- or infectious-type alterations of the sebaceous glands, which encompasses from the simple functional disorder (acne vulgaris) to rosaceous acne, with dilatations of the cutaneous vessels. Acne is characterised by blackheads, outbreaks of pimples, cysts, infected abscesses. Acne may lead to the appearance of severe, permanent scarring. Currently, there are numerous creams, gels, oils and lotions in the market designed for the treatment of acne; however, although they provide a slight improvement whilst being applied, these products do not represent a definitive solution that allows for the elimination of acne and the irritations and infections produced by the different varieties of acne.

In this regard, it is worth noting the existence of Spanish Patent 200400524 (ES2238937), from the same holder of the present invention, which discloses a natural chemical liquid composition against infections that contains totally natural substances, including Chamomilla recutita and Matricaria chamomilla, commonly known as ecological chamomile, natural mineral water and 70° alcohol boricated to saturation. The liquid obtained does not present any secondary or collateral effects and, in and of itself, represents an evident solution for any type of infection, since the combination of said products makes it possible for users to receive an integral treatment that heals any type of irritation or infection, especially buccal infections, infections caused by chronic otitis.

However, the holder of the present invention is not aware of the existence of any products that provide wholly satisfactory, definitive results in the treatment of acne and the irritations and infections produced by the different varieties of acne.

### Description of the invention

The cream of this invention represents an effective solution for the treatment of acne and the irritations and infections produced by the different varieties of acne, and is applicable to human beings regardless of their sex and age, with the particularity that it may be used either as a shaving cream or as a face mask.

This cream does not present any secondary or collateral effects and, in and of itself, represents a solution for any type of irritation or infection caused by the different types of acne.

The cream of the invention is composed of a mixture of a solid part and a liquid part, in a proportion of 1 gram of solid part for every 2.65 millilitres of liquid part.

According to the invention, the solid part is composed of sweet almond powdered soap, rich in moisturising glycerine, whereas the liquid part includes the following components: orchid gel, sweet almond oil, hypoallergenic bath gel, ecological chamomile tea, alcohol-free eau de cologne and 70° alcohol boricated to saturation.

The cream's components provide combined effects due to the properties thereof. Specifically, the sweet almond powdered soap, naturally rich in moisturising glycerine, has well-known anti-drying skin properties.

The orchid gel, combined with the soap's detersive properties, increases the regeneration capacity of skin cells.

The ecological almond oil, on the one hand, moisturises skin against dryness and roughness, and, on the other hand, utilises the skin regeneration effects of sweet almonds, as indicated above.

The high-foam, soap-free hypoallergenic gel influences the soap's detersive capacity, and prevents potential allergic reactions in the skin, which could adversely affect healing.

The ecological chamomile tea contributes significant antiseptic effects for the treatment of rashes, abrasions and wounds.

The alcohol-free eau de cologne softens and refreshes the skin, preventing the interactions in the skin caused by the alcohol habitually used in cosmetics.

The 70° alcohol boricated to saturation, combined with the ecological chamomile tea, increases the healing and disinfectant properties of the cream in this product.

Furthermore, it is worth noting that the combination of the ecological chamomile tea, the alcohol-free eau de cologne and the 70° alcohol boricated to saturation provide the cream with the necessary humidity so that it remains fresh and moist through time.

In sum, the cream components provide a combination of disinfectant, skin regeneration, anti-inflammatory and antiseptic effects; said components, in the adequate proportions, provide the cream with a surprising healing effect for the treatment of acne and the irritations and infections produced by the different varieties of acne, leaving no trace whatsoever of this condition.

The mode of application required to cure acne and the irritations and infections produced by the different varieties of acne is as follows: A fine-hair brush is moistened with natural mineral water; subsequently, a quantity of 0.5 grams (approximately a corn seed) of the shaving cream of the present invention is placed on the brush; thereafter, the acne-affected area is rubbed for 2 to 3 minutes and allowed to dry for another 2 minutes. In the case of men, if they need to shave, the area will have to be moistened once again before shaving. In the case of women, the same process is followed as in the case of men, but the face is washed after the 2 minutes have elapsed. In the remaining parts of the body, both in the case of men and women, the treatment to be followed is always the same, as explained for the case of women.

The use of a moistened fine-hair brush to apply and rub the cream in the acne areas produces a massage in these areas that is very beneficial for the treatment, since it facilitates interaction of the cream with the skin. The treatment to be followed may last from 4 days to several months, depending on the type of acne to be treated. As mentioned above, once the acne is cured, no traces of the acne suffered are observed.

### PREFERRED EMBODIMENT OF THE INVENTION

Thus, in a specific embodiment, the cream is composed of a proportion of 1 gram of solid part for every 2.65 millilitres of liquid part; the solid part is composed of powdered soap and the liquid part is composed of a combination of the following components: orchid gel, sweet almond oil, hypoallergenic bath gel, ecological chamomile tea, alcohol-free eau de cologne and 70° alcohol boricated to saturation.

In a specific embodiment, the cream composition includes the aforementioned components, in the quantities listed below:
100 grams of sweet almond powdered soap, rich in moisturising glycerine, 100 millilitres of orchid gel,
20 millilitres of almond oil,
40 millilitres of high-foam, soap-free baby bath gel,
50 millilitres of ecological chamomile tea,
50 millilitres of alcohol-free baby eau de cologne, and
5 millilitres of 70° alcohol boricated to saturation.

Having sufficiently described the invention, as well as a preferred embodiment example, we state, to all appropriate effects, that the components and proportions thereof may be modified, provided that this does not entail an alteration of the essential characteristics of the invention claimed below.

## Claims

1. Shaving cream against acne and the irritations and infections caused by acne, **characterised in that** it is composed of a mixture of a solid part and a liquid part, with a proportion of 1 gram of solid part for every 2.65 millilitres of liquid part; where the solid part is composed of sweet almond powdered soap, rich in moisturising glycerine; and the liquid part is composed of a combination of the following components: orchid gel, almond oil, high-foam, soap-free baby bath gel, ecological chamomile tea, alcohol-free baby eau de cologne and 70° alcohol boricated to saturation.

2. Shaving cream against acne and the irritations and infections caused by acne according to claim 1, **characterised in that** the quantities of the liquid part components corresponding to 100 grams of the solid part are as follows: 100 millilitres of orchid gel, 20 millilitres of almond oil, 40 millilitres of high-foam, soap-free baby bath gel, 50 millilitres of ecological chamomile tea, 50 millilitres of alcohol-free baby eau de cologne, and 5 millilitres of 70° alcohol boricated to saturation.

3. Shaving cream against acne and the irritations and infections caused by acne according to claims 1 and 2, **characterised in that** it may be presented as a shaving cream or as a face mask.
